# EUROPEAN PATENT APPLICATION

(11) **EP 4 497 834 A1**
(43) Date of publication of application: **29.01.2025**
(21) Application number: 23774412.3
(22) Date of filing: 01.03.2023
(51) Int. Cl.: C12Q 1/6844, C12Q 1/6851, C12Q 1/6888, C12Q 1/70, C12M 1/00

(54) **NUCLEIC ACID AMPLIFICATION METHOD, NUCLEIC ACID AMPLIFICATION SYSTEM, VIRUS DETECTION METHOD, AND VIRUS DETECTION SYSTEM**

(30) Priority: 22.03.2022 JP 2022045736
(71) Applicant: Kyocera Corporation, Kyoto-shi, Kyoto 612-8501 (JP)
(72) Inventor: SATO, Masaki, Kyoto-shi, Kyoto 612-8501 (JP); KURIHARA, Kensuke, Kyoto-shi, Kyoto 612-8501 (JP)
(74) Representative: TBK
(86) International application number: PCT/JP2023/007563
(87) International publication number: WO 2023/181813

(57) **Abstract**

To achieve a nucleic acid amplification method and a nucleic acid amplification system capable of simply amplifying a nucleic acid in a short period of time, and a virus detection method and a virus detection system. The nucleic acid amplification method includes: a first preparation step of preparing a first liquid by adding a nonionic surfactant, dNTPs, and NTPs to a specimen containing a virus; a first incubation step of retaining the first liquid in a first temperature zone for a first period of time; a second preparation step of preparing a second liquid by adding a first primer corresponding to a first region of a nucleic acid molecule derived from the virus, a second primer corresponding to a second region, and an enzyme to the first liquid after the first incubation step; and a second incubation step of retaining the second liquid in a second temperature zone for a second period of time.

## Description

### TECHNICAL FIELD

The present disclosure relates to a nucleic acid amplification method, a nucleic acid amplification system, and a virus detection method and a virus detection system using the same.

### BACKGROUND OF INVENTION

In a method of detecting bacteria, viruses, and the like contained in a specimen, nucleic acids are extracted from the specimen; a target nucleic acid containing a specific sequence, among the extracted nucleic acids, is amplified; and detection is performed. When the target nucleic acid is RNA, for example, the Nucleic A1cid Sequence-Based Amplification (NASBA) method is applied (Non-Patent Document 1).

### CITATION LIST

### NON-PATENT LITERATURE

Non-Patent Document 1: Compton, J. "Nucleic acid sequence-based amplification." Nature 350, 91-92, 1991.

### SUMMARY

A nucleic acid amplification method according to an aspect of the present disclosure includes:
a first preparation step of preparing a first liquid by adding a nonionic surfactant, dNTPs, and NTPs to a specimen containing a virus;
a first incubation step of retaining the first liquid in a first temperature zone for a first period of time;
a second preparation step of preparing a second liquid by adding, after the first incubation step, a first primer corresponding to a first region of a nucleic acid molecule derived from the virus, a second primer corresponding to a second region different from the first region, and an enzyme to the first liquid; and
a second incubation step of retaining the second liquid in a second temperature zone for a second period of time.

A nucleic acid amplification system according to an aspect of the present disclosure includes an amplifier and a temperature control device. The amplifier includes: a first preparator that prepares a first liquid by adding a nonionic surfactant, dNTPs, and NTPs to a specimen containing a virus; a first incubator that retains the first liquid in a first temperature zone for a first period of time; a second preparator that prepares a second liquid by adding a first primer corresponding to a first region of a predetermined nucleic acid molecule, a second primer corresponding to a second region different from the first region, and an enzyme to the first liquid; and a second incubator that retains the second liquid in a second temperature zone for a second period of time. The temperature control device includes: an installation section that installs the amplifier; and a temperature controller that controls temperatures of the first incubator and the second incubator.

A virus detection system according to an aspect of the present disclosure includes an amplifier and a detection device. The amplifier further includes: a first preparator that prepares a first liquid by adding a nonionic surfactant, dNTPs, and NTPs to a specimen containing a virus; a first incubator that retains the first liquid in a first temperature zone for a first period of time; a second preparator that prepares a second liquid by adding a first primer corresponding to a first region of the nucleic acid molecule derived from the virus, a second primer corresponding to a second region different from the first region, and an enzyme to the first liquid; a second incubator that retains the second liquid in a second temperature zone for a second period of time; and a detection preparation section that adds a probe DNA containing a predetermined base sequence and capable of binding to the nucleic acid molecule amplified by the enzyme to the second liquid. The detection device includes: an installation section that installs the amplifier; a temperature controller that controls temperatures of the first incubator and the second incubator; and a detector that detects the nucleic acid molecule amplified by the enzyme based on intensity of fluorescence emitted from the probe DNA. Each molecule of the probe DNA is modified with a fluorescent molecule and a quenching molecule. A distance between the fluorescent molecule and the quenching molecule in the probe DNA not bound to the nucleic acid molecule amplified by the enzyme is shorter than a distance between the fluorescent molecule and the quenching molecule in the probe DNA bound to the nucleic acid molecule amplified by the enzyme.

### BRIEF DESCRIPTION OF THE DRAWINGS

FIG. 1 illustrates an outline of a diagnosis system according to an embodiment of the present disclosure.
FIG. 2 is an external view illustrating an example of a nucleic acid amplification system according to an embodiment of the present disclosure.
FIG. 3 is a block diagram illustrating a configuration of a virus detection system according to an embodiment of the present disclosure.
FIG. 4 is a flowchart illustrating a flow of a nucleic acid amplification method and a virus detection method.
FIG. 5 illustrates fluorescence micrographs of mixtures of respective viruses and molecular beacons.
FIG. 6 is a bar graph illustrating fluorescence intensity.

### DESCRIPTION OF EMBODIMENTS

### Embodiment

An embodiment of the present disclosure will be described in detail below.

### Technical Idea of Present Disclosure

The use of the nucleic acid amplification method and the nucleic acid amplification system according to an aspect of the present disclosure makes it possible to readily extract and amplify a nucleic acid contained in a collected specimen in a short period of time. Therefore, the virus detection method and the virus detection system using the nucleic acid amplification method and the nucleic acid amplification system can readily detect bacteria, viruses, and the like contained in a specimen in a short period of time with high sensitivity. Here, the specimen may be saliva, urine, sweat, nasal discharge, blood, cells, or the like collected from the body of a subject, or may be, for example, earth and sand, water, or the like collected from a test target such as the ground, a river, or the sea. Alternatively, the specimen may be an adherent collected from a surface of a test target such as a handrail, a door, clothes, shoes, or a toilet bowl. The type of virus contained in the specimen is not limited to one type, and may be two or more types. Examples of the type of virus include influenza virus, coronavirus (e.g., SARS-CoV-2), RS virus, human metapneumovirus, norovirus, HIV virus, and herpes virus.

Hereinafter, a diagnosis system 1 that detects a virus contained in a specimen by applying a nucleic acid amplification method and a nucleic acid amplification system according to an embodiment of the present disclosure will be described as an example.

### Configuration of Diagnosis System

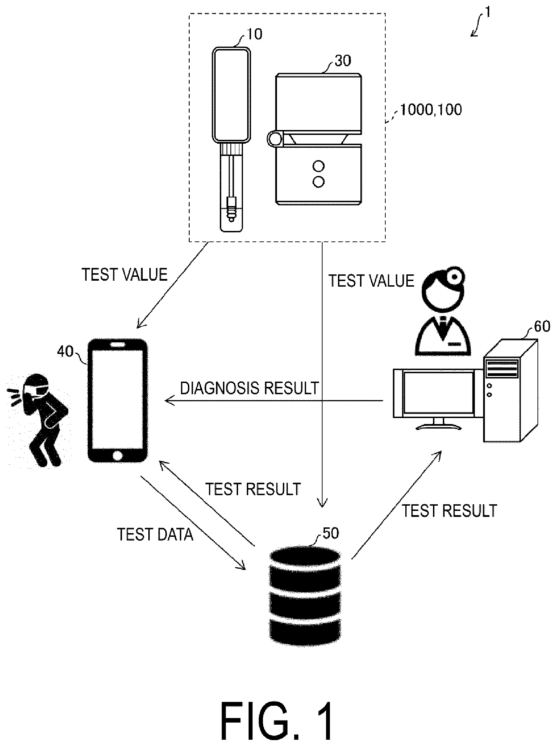

FIG. 1 is a diagram illustrating an outline of a diagnosis system 1 according to an embodiment of the present disclosure. As illustrated in FIG. 1, the diagnosis system 1 includes a virus detection system 1000, a subject's terminal device 40, a server 50, and a medical person's terminal device 60. The virus detection system 1000 includes an amplifier 10 and a detection device 30. The terminal device 40 and the server 50 may be communicably connected to each other.

The amplifier 10 has a function as a container that extracts a nucleic acid from a collected specimen collected from a subject (for example, saliva, urine, blood, or the like of the subject) and to amplify a nucleic acid to be detected. The amplifier 10 may be, for example, a disposable type cartridge.

On the other hand, the detection device 30 includes an installation section 31 for installing the amplifier 10, a temperature controller 32 that controls the temperature control device 20 that controls a temperature of the installed amplifier 10, and a detector 33 that detects a target nucleic acid amplified by the installed amplifier 10. The installation section 31, the temperature controller 32, and the detector 33 will be described later. For example, when the target nucleic acid is detected by fluorescent labeling of the target nucleic acid, the detection device 30 functions as a fluorescence microscope capable of measuring fluorescence intensity. As an example, the detection device 30 may output the measured fluorescence intensity as a detection value. The detection device 30 may include a communicator (not illustrated) and transmit the detection value to the subject's terminal device 40.

Hereinafter, a system composed of the amplifier 10, the installation section 31 and temperature controller 32 included in the detection device 30, and the temperature control device 20 is referred to as a nucleic acid amplification system 100. That is, the nucleic acid amplification system 100 refers to a portion obtained by removing the detector 33 included in the detection device 30 from the virus detection system 1000.

The terminal device 40 may output an analysis result obtained by analyzing the detection value acquired from the detection device 30 by using, for example, an application to the server 50. In this case, the server 50 may store the analysis result for each subject. Alternatively, the terminal device 40 may output the detection value acquired from the detection device 30 to the server 50. In this case, the server 50 may analyze the detection value, store the analysis result for each subject, and transmit the analysis result to the terminal device 60 of a medical person in charge of the subject and the subject's terminal device 40.

For example, diagnosis information to which findings are attached by the medical person, may be input in the terminal device 60 that has acquired the analysis result, or the diagnosis information may be transmitted to the subject's terminal device 40.

The server 50 may be a single server, or may include two or more servers. The server 50 may include an analysis server for analyzing test results from test data, and may include a database server for storing analysis results for each test subject for a long period of time.

### Nucleic Acid Amplification System 100

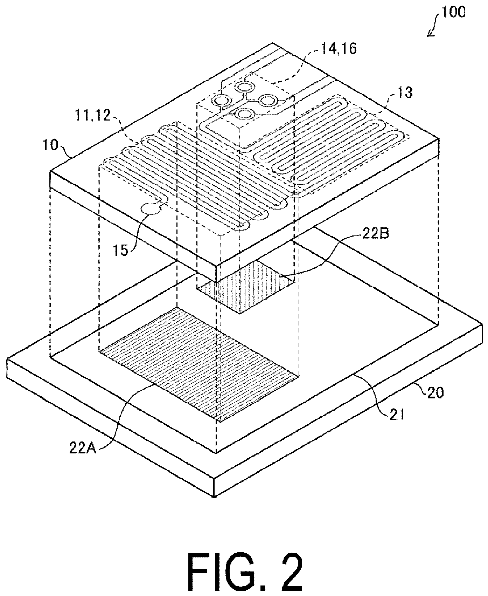

FIG. 2 is an external view illustrating an example of the nucleic acid amplification system 100. In FIG. 2, for simplification of the description, the detection device 30 including the temperature controller 32 that controls the temperature control device 20 is not illustrated.

As illustrated in FIG. 2, the nucleic acid amplification system 100 is a system including the amplifier 10 including a microchannel in which an injection section 15, a first preparator 11, a first incubator 12, a second preparator 13, and a second incubator 14 which will be described below are disposed on a single channel. As a result, the specimen injected into the injection section 15 flows through the microchannel while undergoing various treatments, and reaches the second incubator 14. That is, the microchannel functions as a single reaction system in which a plurality of treatments are performed inside. The number of channels is not limited to one as long as they function as a single reaction system in which a plurality of treatments is performed.

As described above, the amplifier 10 includes the injection section 15, the first preparator 11, the first incubator 12, the second preparator 13, and the second incubator 14.

The injection section 15 is one end portion of the microchannel and is supplied with a specimen containing a virus.

The first preparator 11 is a channel through which the specimen supplied to the injection section 15 flows. The first preparator 11 may be a channel that meanders a plurality of times. The meandering channel promotes stirring of the specimen flowing through the channel. In the first preparator 11, a first liquid is prepared by adding a nonionic surfactant, dNTPs, and NTPs to a specimen containing a virus. The nonionic surfactant may be a surfactant having a repeating structure of ethylene glycol (-O-CH₂-CH₂-), and specifically may be Tween-20 (trade name) or Triton X-100 (trade name).

In the first incubator 12, the first liquid is retained in a first temperature zone for a first period of time. The first period of time during which the first incubator 12 is retained at the first temperature may be shorter than a second period of time during which the second incubator 14 is retained at a second temperature, which will be described later. In this way, the first period of time and the second period of time may be controlled by, for example, lengths of the channels of the first incubator 12 and the second incubator 14, or sizes of temperature control areas 22A and 22B which will be described later.

In the second preparator 13, a second liquid is prepared by adding a first primer corresponding to a first region of a predetermined nucleic acid molecule, a second primer corresponding to a second region different from the first region, and an enzyme to the first liquid, and adding a second liquid thereto. The first primer may be a forward primer that amplifies the nucleic acid molecule to be detected in a sense direction, and the second primer may be a reverse primer that amplifies the nucleic acid molecule in an antisense direction.

The predetermined nucleic acid molecule may be RNA. The enzyme added in the second preparator 13 may include a reverse transcriptase, an RNA polymerase, and a ribonuclease.

In the second incubator 14, the second liquid is retained in a second temperature zone for the second period of time.

The first preparator 11 and the first incubator 12 are installed in the amplifier 10 including channels communicating with each other. Due to this configuration, in the nucleic acid amplification system 100, the first liquid prepared in the first preparator 11 spontaneously reaches the first incubator 12 along the microchannel. Therefore, in the nucleic acid amplification system 100, it is not necessary to transfer the first liquid to a container for incubation.

The first preparator 11, the first incubator 12, the second preparator 13, and the second incubator 14 may be installed in the same amplifier 10 including channels communicating with each other. Due to this configuration, in the nucleic acid amplification system 100, it is unnecessary to take out and transfer each liquid after preparation of the first liquid, after the first retainment of the first liquid in the first temperature zone, after preparation of the second liquid, and after retainment of the second liquid in the second temperature zone. Therefore, each step can be performed in the amplifier 10.

(1) The first primer, (2) the second primer, (3) the enzyme, and (4) a probe DNA containing a predetermined base sequence and capable of binding to the nucleic acid molecule amplified by the enzyme may be immobilized in advance to at least any of the second preparator 13 and the second incubator 14. Due to this configuration, the nucleic acid amplification system 100 can provide each of the first primer, the second primer, the enzyme, and the probe DNA to the second liquid without adding these components later. Hereinafter, the probe DNA is also referred to as a molecular beacon.

For immobilization of the first primer, the second primer, the enzyme, and the probe DNA, a moisture-resistant coating agent may be used, and for example, a saccharide may be used. Examples of the saccharide include trehalose dihydrate, sucrose, lactose, glucose, and fructose. Since the first primer, the second primer, the enzyme, and the probe DNA are immobilized as described above, the nucleic acid amplification system 100 can store each component in the unused amplifier 10 for a long period of time.

Further, the temperature control device 20 may be provided with an installation section 21 for installing the amplifier 10, a temperature control area 22A for controlling the first incubator 12, and a temperature control area 22B for controlling a temperature of the second incubator 14. In this case, the temperature control area 22A is provided at a position corresponding to a lower portion of the first incubator 12, and the temperature control area 22B is provided at a position corresponding to a lower portion of the second incubator 14.

The temperature control device 20 may control the first temperature zone to be a temperature zone higher than the second temperature zone. The first temperature zone may include temperatures for inactivating the virus to be detected, and the second temperature zone may include temperatures for activating the enzyme.

The first temperature zone can be determined according to an amount of the nonionic surfactant added and is not particularly limited. The first temperature zone may be, for example, in a range of 25°C or higher and 100°C or lower. More specifically, the first temperature zone may be in a range of 95°C or higher and 100°C or lower, and the second temperature zone may be in a range of 40°C or higher and 42°C or lower.

The first temperature zone and the second temperature zone may include temperatures set by the temperature control device 20. Alternatively, the first temperature zone and the second temperature zone may include a liquid temperature of the liquid to be temperature-controlled by the temperature control device 20. When the first temperature zone and the second temperature zone include the liquid temperature, for example, an average temperature or a center temperature may be used. Here, the average temperature may be, for example, an average of the liquid temperature in a predetermined period of time. The center temperature may be, for example, a temperature at the center between a maximum value and a minimum value of the liquid temperature. The center temperature can be calculated as a sum of the maximum temperature and the minimum temperature divided by two.

### Virus Detection System

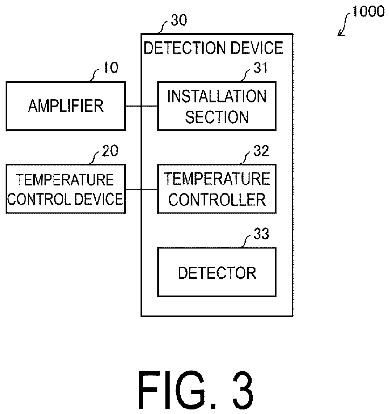

FIG. 3 is a block diagram illustrating a configuration of the virus detection system 1000 according to an embodiment of the present disclosure. As illustrated in FIG. 3, the virus detection system 1000 includes the amplifier 10 and the detection device 30.

As illustrated in FIG. 2, the amplifier 10 includes the first preparator 11, the first incubator 12, the second preparator 13, and the second incubator 14. Here, in the virus detection system 1000, the amplifier 10 may include, in the second incubator 14, a structure (detection preparation section) that adds the probe DNA containing a predetermined base sequence and capable of binding to the nucleic acid molecule amplified by the enzyme to the second liquid. The detection preparation section may be installed at a subsequent stage of the second incubator 14.

The detection preparation section may include, for example, a plurality of reaction fields, and probe DNAs capable of binding to nucleic acid molecules of different viruses may be added to the second liquid in the respective reaction fields. For example, a probe DNA capable of binding to a nucleic acid molecule of an influenza virus and a probe DNA capable of binding to a nucleic acid molecule of a coronavirus (SARS-CoV-2) may be added to the second liquid in the respective reaction fields. This makes it possible to detect a plurality of viruses by one specimen collection.

As described above, the detection device 30 includes the installation section 31, the temperature controller 32, and the detector 33. The temperature controller 32 can individually control the temperatures of the temperature control area 22A and the temperature control area 22B of the temperature control device 20 of FIG. 2.

The detector 33 may have a fluorescence intensity detection function of detecting the nucleic acid molecule amplified by the enzyme based on intensity of fluorescence emitted from the probe DNA. The detector 33 may function as, for example, a fluorescence microscope. The detector 33 may include, for example, an excitation light source for exciting the probe DNA to be measured, and a light reception section that receives fluorescence emitted by the probe DNA in response to the excitation light. Further, the detector 33 may be a camera capable of acquiring an image of fluorescence emission.

Each molecule of the probe DNA is modified with a fluorescent molecule and a quenching molecule. The quenching molecule is set, for example, to absorb light in a wavelength band corresponding to a fluorescence wavelength of the fluorescent molecule. A distance between the fluorescent molecule and the quenching molecule in the probe DNA not bound to the nucleic acid molecule amplified by the enzyme is shorter than a distance between the fluorescent molecule and the quenching molecule in the probe DNA bound to the nucleic acid molecule amplified by the enzyme. Examples of the fluorescent molecule include 6-carboxyfluorescein (FAM), Texas Red (TR), and cyanine (CY)-based materials. The base sequence of the probe DNA may be appropriately designed based on a base sequence of the nucleic acid molecule to be detected.

(1) The first primer, (2) the second primer, (3) the enzyme, and (4) the probe DNA containing a predetermined base sequence and capable of binding to the nucleic acid molecule amplified by the enzyme may be immobilized in advance to at least any of the second preparator 13, the second incubator 14, and the detection preparation section 16. Due to this configuration, the virus detection system 1000 can provide the probe DNA to the second liquid without adding the probe DNA later.

For immobilization of the probe DNA, a moisture-resistant coating agent may be used, and for example, a saccharide may be used. Examples of the saccharide include trehalose dihydrate, sucrose, lactose, glucose, and fructose. The immobilized probe DNA enables the detector 33 to detect fluorescence at a fixed position.

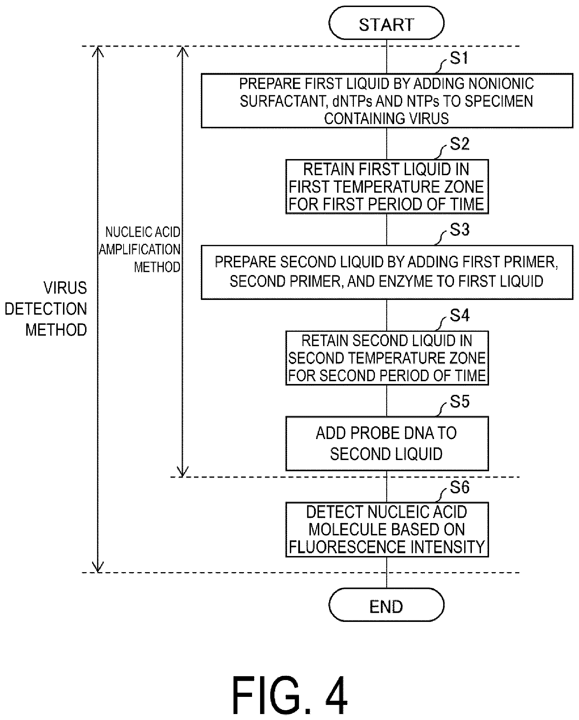

Nucleic Acid Amplification Method and Virus Detection Method The nucleic acid amplification method and the virus detection method will be described with reference to FIG. 4. FIG. 4 is a flowchart illustrating a flow of the nucleic acid amplification method and the virus detection method.

In S1, the first preparator 11 prepares a first liquid by adding a nonionic surfactant, dNTPs, and NTPs to a specimen containing a virus (first preparation step). The nonionic surfactant may be a surfactant having a repeating structure of ethylene glycol (-O-CH₂-CH₂-), and specifically may be Tween-20 (trade name) or Triton X-100 (trade name).

Next, in S2, the first incubator 12 retains the first liquid in a first temperature zone for a first period of time (first incubation step). The first temperature zone may be a temperature zone higher than the second temperature zone in S4 which will be described later. The first temperature zone can be determined, for example, according to the amount of the nonionic surfactant added and is not particularly limited. The first temperature zone may be, for example, in a range of 25°C or higher and 100°C or lower. More specifically, the first temperature zone may be in a range of 95°C or higher and 100°C or lower, and the second temperature zone may be in a range of 40°C or higher and 42°C or lower. In addition, the first period of time may be shorter than the second period of time in S4 which will be described later.

The first preparation step in S1 and the first incubation step in S2 may be performed in the same reaction system. According to the above configuration, a step of taking out the nucleic acid molecule from a solution in the system to the outside, a step of using the taken out nucleic acid molecule in another system, and the like are not necessary, and the steps are continuously performed. Thus, the nucleic acid can be easily amplified in a short period of time.

In S3, after the first incubation step, the second preparator 13 prepares a second liquid by adding, to the first liquid, a first primer corresponding to a first region of a nucleic acid molecule derived from a virus, a second primer corresponding to a second region different from the first region, and an enzyme (second preparation step). The nucleic acid molecule may be RNA and the enzyme may include a reverse transcriptase, an RNA polymerase, and a ribonuclease. The first primer, the second primer, and the enzyme may be added together with a saccharide.

In S4, the second incubator 14 retains the second liquid in a second temperature zone for a second period of time (second incubation step).

The first preparation step in S1, the first incubation step in S2, the second preparation step in S3, and the second incubation step in S4 may be performed in the same reaction system. The same reaction system may be, for example, an inside of the same channel, or an inside of the same specific region in the channel. According to the above configuration, a step of taking out the nucleic acid molecule from a solution in the system to the outside, a step of using the taken out nucleic acid molecule in another system, and the like are not necessary, and the steps are continuously performed. Thus, the nucleic acid can be easily amplified in a short period of time.

In S5, the second incubator 14 or the detection preparation section 16 adds a probe DNA containing a predetermined base sequence and capable of binding to a nucleic acid molecule amplified by an enzyme to the second liquid (probe DNA addition step). The probe DNA may be added together with a saccharide. Each molecule of the probe DNA is modified with a fluorescent molecule and a quenching molecule. A distance between the fluorescent molecule and the quenching molecule in the probe DNA not bound to the nucleic acid molecule amplified by the enzyme is shorter than a distance between the fluorescent molecule and the quenching molecule in the probe DNA bound to the nucleic acid molecule amplified by the enzyme.

Viral nucleic acid amplification is performed through S1 to S5.

Next, in S6, the nucleic acid molecule amplified by the enzyme in S1 to S5 is detected based on intensity of fluorescence emitted from the fluorescent molecule (detection step).

In the nucleic acid amplification method, a nucleic acid molecule derived from a virus is amplified by retaining, at a second temperature, a second liquid obtained by adding a first primer, a second primer, and an enzyme to a first liquid obtained after adding a nonionic surfactant, dNTPs, and NTPs to a specimen containing a virus and retaining the first liquid at a first temperature. In addition, in the virus detection method, the nucleic acid molecule amplified by the nucleic acid amplification method including S1 to S5 is detected based on the intensity of fluorescence emitted from the fluorescent molecule.

The nucleic acid amplification method including S1 to S5 and the virus detection method including S1 to S6 do not require extraction or purification of a nucleic acid molecule derived from a virus from the first liquid, and make it possible to amplify the virus-derived nucleic acid molecule and detect the virus easily in a short period of time.

The invention according to the present disclosure has been described above based on the drawings and examples. However, the invention according to the present disclosure is not limited to each embodiment described above. That is, the embodiments of the invention according to the present disclosure can be modified in various ways within the scope illustrated in the present disclosure, and embodiments obtained by appropriately combining the technical means disclosed in different embodiments are also included in the technical scope of the invention according to the present disclosure. In other words, a person skilled in the art can easily make various variations or modifications based on the present disclosure. Note that these variations or modifications are included within the scope of the present disclosure.

### EXAMPLE

An example of the present disclosure will be described below.

### Reagents Used

In this example, reagents, enzymes and the like used in the known Nucleic Acid Sequence-Based Amplification (NASBA) method were used.

### Virus

· Influenza A virus (A/Puetro Rico/8/1934 (H1N1), PR8) prepared with reference to the paper [Eisfeld, A. J., 2014]
· Coronavirus RNA (SARS-CoV-2, RNA N1 domain sequence, SEQ ID NO: 1) Kit
· NASBA amplification kit (KAINOS Laboratories, Inc.) Saccharide
· Trehalose dihydrate (available from FUJIFILM Wako Pure Chemical Corporation)
· Sucrose (available from FUJIFILM Wako Pure Chemical Corporation) Surfactant
· Triton X-100 (trade name) (available from FUJIFILM Wako Pure Chemical Corporation)
· Sodium dodecylbenzenesulfonate (SDBS) (available from FUJIFILM Wako Pure Chemical Corporation)
· Tween 20 (available from FUJIFILM Wako Pure Chemical Corporation) Kit
· SARS-CoV-2 Direct Detection RT-qPCR Core Kit (available from Takara Bio Inc.)
· RNase-free water (available from Takara Bio Inc.)

### Others

· Artificial saliva (available from Merck KgaA)
· Primer (whose synthesis was requested to Fasmac)
· Molecular beacon (whose synthesis was requested to Fasmac)

### Designs of Primer and Molecular Beacon

Primers and molecular beacons for influenza A (PR8) virus and for SARS-CoV-2 virus were each designed with reference to a manual disclosed by the National Institute of Infectious Diseases (NIID) (National Institute of Infectious Diseases, 2020). One of primer pairs contains a T7 promoter sequence of SEQ ID NO: 2 in the Sequence Listing at the 5' end. A primer pair for segment 7 of PR8 includes a forward primer 1 of SEQ ID NO: 3 and a reverse primer 2 of SEQ ID NO: 4.

A primer pair for N1 domain RNA of SARS-CoV-2 includes a forward primer 3 of SEQ ID NO: 5 and a reverse primer 4 of SEQ ID NO: 6.

A molecular beacon includes a fluorescent molecule at the 5' end (carboxyfluorescein, FAM, λex = 492 nm, λem = 518 nm) and a quenching molecule corresponding to the fluorescent molecule at the 3' end (black hole quencher 1, BHQ1), or a fluorescent molecule at the 3' end (Texas Red, TR, λex = 596 nm, λem = 615 nm) and a quenching molecule corresponding to the fluorescent molecule at the 5' end (black hole quencher 2, BHQ2). The recognition sequence of the molecular beacon was a 15-base sequence.

A molecular Beacon 1 for PR8 (MB1) is
5'-FAM-CCAAGCCCCTCAAAGCCGAGAGCTTGG-BHQ1-3' (SEQ ID NO: 7). A molecular beacon 2 for PR8 (MB2) is
5'-TR-CCAAGCCCCTCAAAGCCGAGAGCTTGG-BHQ2-3' (SEQ ID NO: 8). The recognition sequences of MB1 and MB2 are the same base sequence. A molecular beacon 3 (MB3) for SARS-CoV-2 is
5'-FAM-CCAAGCACTTCCTCAAGGAACAACATTGCCAGCTTGG-BHQ1-3' (SEQ ID NO: 9).

### Amplification Method

### NASBA Reagent

· Lyophilized ribonucleoside triphosphates (NTPs), Lyophilized deoxyribonucleoside triphosphates (dNTPs)
· Lysis buffer (Tris buffer, DMSO, with metal ions) Enzyme
· Lyophilized AMV-RT
· RnaseH
· T7RNAP

To a 2-mL Eppendorf tube, 27 µL of virus-containing artificial saliva (PR8, 10³ PFU/mL, SARS-CoV-2, from 10⁻¹⁵ M) was added. Subsequently, 3 µL of a 10 wt% Triton X-100 solution was added, and the mixture was incubated for 1 minute. The incubated solution was directly used in the next step without being subjected to a purification step. To a 0.2-mL PCR tube, 10 µL of an NASBA reagent solution, 5 µL of virus-containing artificial saliva, and 5 µL of an enzyme solution were added. They were mixed at room temperature to give 20 µL of an NASBA amplification solution. A specimen for fluorescence microscopic observation was incubated at 41°C for from 0 to 90 minutes.

### Detection of Amplified RNA

A molecular beacon (MB) solution was obtained by dissolving the molecular beacon in 1 µM of Rnase-free water. Ten (10) minutes before the end of the amplification, 20 µL of the NASBA amplification solution and 10 µL of the MB solution were mixed and an amplification reaction was carried out for 10 minutes thereafter.

For the specimen for the amplification reaction for 0 minutes, the reagents other than the enzyme and MB were heated at 41°C for 10 minutes, cooled to room temperature, and then added with the enzyme immediately before microscopic observation.

For the specimen for the amplification reaction for 10 minutes, the enzyme solution and the MB solution were simultaneously added to the specimen, and the mixture was heated at 41°C for 10 minutes.

The amplified RNA mixture was installed together with a spacer (17 × 28 mm, 0.3 mm thick, In situ PCR Frame-Seal Incubation Chamber, available from Bio-Rad Laboratories) between two glass coverslips (24 × 60 mm, from 0.13 to 0.17 mm thick, available from MATSUNAMI).

The specimen was observed by a fluorescent microscope (IX83, available from Olympus) equipped with an LED emitter (X-Cite XYLIS, available from Excelitas Technologies) and an optical filter set (IX3-FGFPXL; λex: from 460 to 480 nm, λem: from 495 to 540 nm, U-RFP; λex: from 535 to 555 nm, λem: from 570 to 625 nm, available from Olympus). Images were captured by imaging software (cellSens Dimension 2.3, available from Olympus) and analyzed by ImageJ software (version 1.53e, NIH).

### Detection of Virus

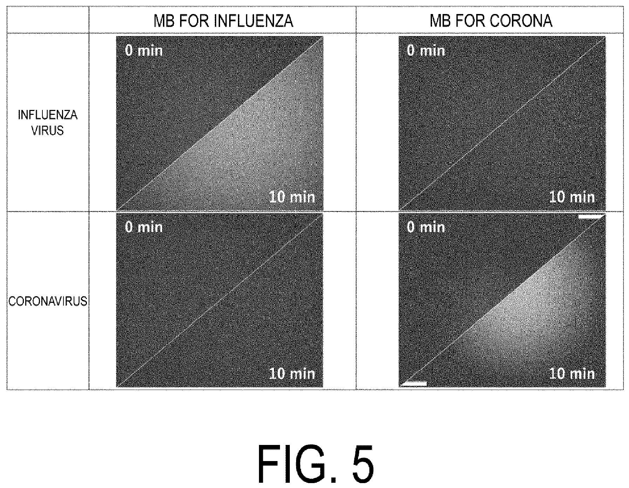

The upper part of FIG. 5 illustrates fluorescent micrographs of influenza virus obtained by mixing MB1 for influenza (left panel) and MB3 for SARS-CoV-2 (right panel) and performing an amplification reaction. The lower part of FIG. 5 illustrates fluorescent micrographs of SARS-CoV-2RNA obtained by mixing MB1 (left panel) and MB3 (right panel) and performing an amplification reaction. Each micrograph includes images for the amplification reactions for 0 minutes and 10 minutes. Influenza virus and coronavirus RNAs were detected only by the corresponding molecular beacons (MBs).

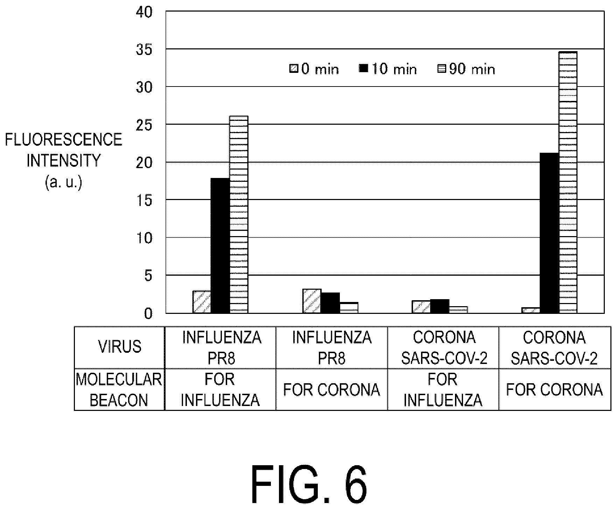

FIG. 6 is a bar graph illustrating numerical values of the fluorescence intensity of FIG. 5. The horizontal axis represents the types of virus or RNA and the corresponding molecular beacon, and the respective amplification times, and the vertical axis represents the fluorescence intensity. FIG. 6 shows that the molecular beacon specifically detects the corresponding virus or RNA. In addition, it was found that the amplification time of 10 minutes was sufficient for the molecular beacon to successfully detect the virus or RNA. From the above, it has been found that the virus detection method of the present disclosure makes it possible to collect saliva and detect from which virus the collected saliva suffers, within 10 minutes.

### REFERENCE SIGNS

10: Amplifier
11: First preparator
12: First incubator
13: Second preparator
14: Second incubator
16: Detection preparation section
20: Temperature control device
21, 31: Installation section
22A, 22B: Temperature control area
30: Detection device
32: Temperature controller
33: Detector
100: Nucleic acid amplification system
1000: Virus detection system

## Claims

1. A nucleic acid amplification method, comprising:
a first preparation step of preparing a first liquid by adding a nonionic surfactant, dNTPs, and NTPs to a specimen containing a virus;
a first incubation step of retaining the first liquid in a first temperature zone for a first period of time;
a second preparation step of preparing a second liquid by adding, after the first incubation step, a first primer corresponding to a first region of a nucleic acid molecule derived from the virus, a second primer corresponding to a second region different from the first region, and an enzyme to the first liquid; and
a second incubation step of retaining the second liquid in a second temperature zone for a second period of time.

2. The nucleic acid amplification method according to claim 1, wherein the first preparation step and the first incubation step are performed in the same reaction system.

3. The nucleic acid amplification method according to claim 2, wherein the first preparation step, the first incubation step, the second preparation step, and the second incubation step are performed in the same reaction system.

4. The nucleic acid amplification method according to any one of claims 1 to 3, wherein the nonionic surfactant is Tween-20 (trade name) or Triton X-100 (trade name).

5. The nucleic acid amplification method according to any one of claims 1 to 4, wherein the first temperature zone is a higher temperature zone than the second temperature zone.

6. The nucleic acid amplification method according to claim 5, wherein the first temperature zone is in a range of 25°C or higher and 100°C or lower.

7. The nucleic acid amplification method according to claim 6, wherein
the first temperature zone is in a range of 95°C or higher and 100°C or lower, and
the second temperature zone is in a range of 40°C or higher and 42°C or lower.

8. The nucleic acid amplification method according to any one of claims 1 to 7, wherein the first period of time is shorter than the second period of time.

9. The nucleic acid amplification method according to any one of claims 1 to 8, wherein
the nucleic acid molecule is RNA; and
the enzyme comprises a reverse transcriptase, an RNA polymerase, and a ribonuclease.

10. The nucleic acid amplification method according to any one of claims 1 to 9, further comprising a probe DNA addition step of adding a probe DNA containing a predetermined base sequence and capable of binding to the nucleic acid molecule amplified by the enzyme to the second liquid, each molecule of the probe DNA being modified with a fluorescent molecule and a quenching molecule,
wherein a distance between the fluorescent molecule and the quenching molecule in the probe DNA not bound to the nucleic acid molecule amplified by the enzyme is shorter than a distance between the fluorescent molecule and the quenching molecule in the probe DNA bound to the nucleic acid molecule amplified by the enzyme.

11. The nucleic acid amplification method according to claim 10, wherein the first primer, the second primer, the enzyme, and the probe DNA are added together with a saccharide.

12. A virus detection method, comprising the nucleic acid amplification method according to claim 10 or 11, and further comprising a detection step of detecting the nucleic acid molecule amplified by the enzyme based on intensity of fluorescence emitted from the fluorescent molecule.

13. A nucleic acid amplification system, comprising an amplifier and a temperature control device,
the amplifier comprising:
a first preparator configured to prepare a first liquid by adding a nonionic surfactant, dNTPs, and NTPs to a specimen containing a virus;
a first incubator configured to retain the first liquid in a first temperature zone for a first period of time;
a second preparator configured to prepare a second liquid by adding a first primer corresponding to a first region of a predetermined nucleic acid molecule, a second primer corresponding to a second region different from the first region, and an enzyme to the first liquid; and
a second incubator configured to retain the second liquid in a second temperature zone for a second period of time, and
the temperature control device comprising:
an installation section configured to install the amplifier; and
a temperature controller configured to control temperatures of the first incubator and the second incubator.

14. The nucleic acid amplification system according to claim 13, wherein the first preparator and the first incubator are installed in the amplifier including channels communicating with each other.

15. The nucleic acid amplification system according to claim 14, wherein the first preparator, the first incubator, the second preparator, and the second incubator are installed in the same amplifier including channels communicating with each other.

16. The nucleic acid amplification system according to any one of claims 13 to 15, wherein (1) the first primer, (2) the second primer, (3) the enzyme, and (4) a probe DNA containing a predetermined base sequence and capable of binding to the nucleic acid molecule amplified by the enzyme are immobilized in advance to at least any of the second preparator and the second incubator.

17. The nucleic acid amplification system according to claim 16, wherein a saccharide is used in the immobilization.

18. A virus detection system, comprising an amplifier and a detection device,
the amplifier further comprising:
a first preparator configured to prepare a first liquid by adding a nonionic surfactant, dNTPs, and NTPs to a specimen containing a virus;
a first incubator configured to retain the first liquid in a first temperature zone for a first period of time;
a second preparator configured to prepare a second liquid by adding a first primer corresponding to a first region of the nucleic acid molecule derived from the virus, a second primer corresponding to a second region different from the first region, and an enzyme to the first liquid;
a second incubator configured to retain the second liquid in a second temperature zone for a second period of time; and
a detection preparation section configured to add a probe DNA containing a predetermined base sequence and capable of binding to the nucleic acid molecule amplified by the enzyme to the second liquid, and
the detection device comprising:
an installation section configured to install the amplifier;
a temperature controller configured to control temperatures of the first incubator and the second incubator; and
a detector configured to detect the nucleic acid molecule amplified by the enzyme based on intensity of fluorescence emitted from the probe DNA,
each molecule of the probe DNA being modified with a fluorescent molecule and a quenching molecule,
wherein a distance between the fluorescent molecule and the quenching molecule in the probe DNA not bound to the nucleic acid molecule amplified by the enzyme is shorter than a distance between the fluorescent molecule and the quenching molecule in the probe DNA bound to the nucleic acid molecule amplified by the enzyme.

19. The virus detection system according to claim 18, wherein (1) the first primer, (2) the second primer, (3) the enzyme, and (4) the probe DNA containing a predetermined base sequence and capable of binding to a nucleic acid molecule amplified by the enzyme are immobilized in advance to at least any of the second preparator, the second incubator, and the detection preparation section.

20. The virus detection system according to claim 19, wherein a saccharide is used in the immobilization.
